# FASCICULE DE BREVET EUROPEEN

(11) **EP 1 068 240 B1**
(45) Date de publication et mention de la délivrance du brevet: **18.12.2002**
(21) Numéro de dépôt: 99913360.6
(22) Date de dépôt: 09.04.1999
(51) Int. Cl.: C07K 14/78, C07K 1/36, A61K 38/39, A61K 9/08, A61K 9/12, A61K 9/14, A61K 7/00, A61L 15/32, A61F 13/02

(54) **PROCEDE DE STERILISATION D'UN COLLAGENE NATIF EN MILIEU LIQUIDE, COLLAGENE NATIF STERILE OBTENU, COMPOSITIONS LE CONTENANT ET APPLICATIONS**
VERFAHREN ZUR STERILISATION VON NATIVEM KOLLAGEN IN EINEM FLÜSSIGMEDIUM, STERILE NATIVE KOLLAGEN ZUSAMMENSETZUNGEN UND VERWENDUNG DAVON
METHOD FOR STERILISING NATIVE COLLAGEN IN LIQUID MEDIUM, RESULTING NATIVE COLLAGEN, COMPOSITIONS CONTAINING SAME AND USES

(30) Priorité: 10.04.1998 FR 9804531
(43) Date de publication de la demande: 17.01.2001
(73) Titulaire: Octapharma AG, 8853 Lachen (CH)
(72) Inventeur: Mansour, Hamza, 67380 Lingolsheim (FR)
(74) Mandataire: Kohn, Philippe
(86) Numéro de dépôt international: FR9900814
(87) Numéro de publication internationale: WO99052946

(56) Documents cités:
- EP-A- 0 214 035
- EP-A- 0 224 453
- EP-A- 0 664 132
- EP-A- 0 681 863
- DE-A- 2 462 222
- DE-A- 3 522 626
- US-A- 3 949 073
- US-A- 5 670 369

## Description

La présente invention a trait à un procédé de préparation de collagène comportant une étape de stérilisation dudit collagène, au collagène stérile ainsi obtenu, aux compositions et applications notamment pharmaceutiques et/ou para-pharmaceutiques et/ou médico-chirurgicales et/ou ophtalmologiques et/ou cosmétiques de ce dernier.

Le collagène et ses applications ont été décrits de manière générale dans des ouvrages de référence tels que Collagen, vol.1, 2, 3 Marcel E. Nimni., CRC Press Inc, 1988 et Methods in Enzymology, vol.144, 1987 et vol.82, 1982., Ed. Leon W. Gunningham., par exemple.

Il est bien connu que le collagène est une molécule à structure tridimensionnelle présente dans la plupart des tissus humains et animaux et se trouve en particulier dans la peau, les tendons et le placenta.

Ses différentes propriétés connues sont, parmi d'autres, l'hydratation, la coagulation du sang et la cicatrisation de plaies.

Pour préserver son action hémostatique, il est en général souhaitable que le collagène conserve son état natif avant son administration dans le corps humain comme rappelé, par exemple, dans le document US-A-4 515 637.

Par ailleurs en raison de ses utilisations, notamment médicales et/ou cosmétiques, le collagène doit être exempt de toute contamination (virus, bactérie, prion etc.). La stérilisation du collagène a été diversement traitée dans l'art antérieur confronté depuis quelques années à de nouveaux défis (risques de transmission du SIDA, de la maladie de Creutzfeldt-Jakob etc., du fait de l'origine naturelle du collagène).

Des techniques de stérilisation du collagène à l'état solide comprennent l'utilisation d'oxyde d'éthylène selon, notamment, le document FR-A-2.393.581 ou l'irradiation (application de rayonnements ionisants bêta ou gamma), comme décrit par exemple dans le document EP-A-0.224.453. Ce dernier, qui décrit des utilisations cosmétiques et pharmaceutiques de type éponge à base de collagène, préconise la stérilisation à l'état sec en raison de l'impossibilité, d'après ce document, de stériliser le collagène naturel soluble incorporé dans des émulsions ou solutions.

Le document EP-A-0.664.132 décrit une composition adhésive, à usage chirurgical, à base de collagène modifié par coupure oxydative et non réticulé. Un mode de réalisation préféré de la composition décrite dans ce document consiste à préparer une poudre stérile de collagène de type I par coupure oxydative, non réticulé et à dissoudre dans de l'eau ultrafiltrée stérile cette poudre en chauffant à environ 60 °C sous agitation. Il est précisé que compte tenu du chauffage contrôlé opéré, le collagène perd sa structure hélicoïdale et se transforme en gélatine. Par ailleurs il est exposé que les compositions adhésives représentant l'état de la technique antérieure de ce document, qui sont à base de collagène réticulé, ne sont pas de manipulation aisée et posent des problèmes d'application et de résistance mécanique.

Le document EP-A-0.667.352 décrit un traitement alcalin appliqué à des collagènes en solution en vue d'éliminer des éventuels prions. Afin d'éviter tout risque de contamination résiduelle par lesdits prions, ce document enseigne plus précisément un procédé consistant à solubiliser le collagène extrait de tissus soit par digestion enzymatique soit par coupure alcaline des liaisons covalentes reliant les chaînes de collagène entre elles puis à éliminer les débris tissulaires par filtration et à faire subir un traitement alcalin au collagène ainsi solubilisé.

Le document FR-A-2.586.703 enseigne la préparation de gels transparents et physiologiques ou de solutions collagéniques à base de collagène de type IV. Il est toutefois rappelé que les collagènes de type I, II ou III, n'étant pratiquement pas solubles à pH neutre, ne permettent que l'obtention de suspensions opaques et qu'il est impossible de les utiliser pour préparer des gels transparents et physiologiques sans recourir à une modification chimique des molécules de collagène susceptibles de les rendre antigéniques et/ou toxiques.

La connaissance de cet art antérieur et la réalisation de travaux de recherche ont permis au présent inventeur de mettre en évidence les difficultés et les problèmes suivants subsistant encore à ce jour dans la préparation de collagène natif stérile, notamment de type I :
* manipulation
   - viscosité élevée du collagène natif, imposant des contraintes techniques pour la réalisation de filtrations, notamment lors d'opérations de filtration directe de collagène classique natif en milieu liquide.
* dénaturation
   - dénaturation thermique partielle ou totale du collagène natif lors des différents traitements stérilisants habituels, entraînant la perte de tout ou partie de sa structure et / ou de ses propriétés bénéfiques, telles que résorption, résistance mécanique ou hydratation, par exemple.
   - stérilisation par irradiation (rayonnements ionisants), ou à l'oxyde d'éthylène causant la détérioration de principes actifs d'intérêt à associer en solution au collagène tels que des antibiotiques, par exemple et/ou provoquant une séparation en deux phases d'un gel classique à base de collagène natif le rendant impropre à une utilisation commerciale .
   - dénaturation par traitement alcalin prolongé
* toxicité et/ou de contamination (virus, prions)
   - présence de traces d'oxyde d'éthylène résiduel dans les produits stérilisés par ce moyen / présence de contaminants dans le collagène brut de départ
* incompatibilité
   - incompatibilité de pH entre des principes actifs d'intérêt solubles à pH neutre, tels que des agents anti-infectieux, par exemple et le collagène soluble en milieu acide.
* galénique
   - présentation insatisfaisante des préparations à base de collagène natif à l'aspect fibreux et hétérogène
   - présentation des préparations classiques de colle biologique inadaptée étant donné l'usage auquel elles sont destinées (telles que des préparations non prêtes à l'emploi et nécessitant un temps de préparation trop long, par exemple).

Aussi le but de la présente invention est de surmonter les difficultés précitées et de résoudre les problèmes mentionnés ci-dessus, notamment la stérilisation du collagène en milieu liquide tout en préservant son état natif, ainsi que d'autres problèmes qui vont être exposés ci-après.

La présente invention a donc pour objet un procédé de préparation de collagène à l'état natif, comportant une étape de prétraitement du collagène dans un mélangeur à double couteaux transversaux équipé d'un système de contrôle de la vitesse d'agitation et de cisaillement ainsi que d'un thermostat, et une étape subséquente de stérilisation du collagène en milieu liquide.

La présente invention a également pour objet un collagène stérile, en particulier un collagène majoritairement de type I, à l'état natif ainsi que des compositions à usage humain et / ou vétérinaire le renfermant en tant que seul principe actif ou dans lesquelles il est associé à d'autres substances d'intérêt acceptables d'un point de vue pharmaceutique, para-pharmaceutique, médico-chirurgical, et / ou ophtalmologique et / ou cosmétique.

Elle a aussi pour objet l'utilisation du collagène conforme à l'invention dans la fabrication d'une composition destinée à un traitement pharmaceutique et / ou para-pharmaceutique et / ou médico-chirurgical et / ou ophtalmologique et / ou cosmétique d'un corps humain ou animal.

Ces objets ainsi que d'autres vont apparaître à l'homme du métier à la lecture de la description détaillée qui suit.

Les procédés de préparation de collagène stérile actuellement disponibles posent les problèmes de mise en oeuvre déjà évoqués ci-dessus et, n'ont pas permis à ce jour, dans la pratique, une stérilisation directe du collagène en milieu liquide seul, ou en présence d'autres principes actifs, qui soit simple à réaliser et surtout qui garantisse effectivement la stérilité, et le caractère natif du collagène obtenu, préservant ainsi ses propriétés bénéfiques.

Aussi la présente invention a atteint cet objectif et concerne un procédé de préparation de collagène, comprenant une étape, préalable à la stérilisation, de prétraitement mécanique, dans un mélangeur -tel qu'un « cutter mélangeur » commercialisé sous la marque DITO-SAMA 175 (LA BOVIDA), modifié par les soins de la société J.F.I. Graulhet, France, par exemple- et équipé d'un système de contrôle de la température, d'un extrait de collagène natif non stérile solubilisé et purifié, éventuellement pepsiné. Cette étape de prétraitement permet, de manière tout à fait surprenante et inattendue, la stérilisation à l'état liquide du collagène et la préservation de son état natif lorsqu'il n'a pas été pepsiné.

De préférence le collagène ainsi préparé est un collagène majoritairement de type I.

Le collagène majoritairement de type I ainsi préparé est stérile et à l'état natif, non dénaturé, c'est à dire ayant ses structures secondaire et tertiaire inchangées et ayant conservé l'ensemble de ses propriétés bénéfiques même après stérilisation.

Le collagène conforme à la présente invention est utile dans de très nombreuses applications détaillées plus loin, notamment :
- médicales humaines et vétérinaires :
   pharmaceutiques
   para-pharmaceutiques
   ophtalmologiques
   médico-chirurgicales
- cosmétiques

Il peut être utilisé tel quel, c'est à dire sans autre principe actif, par exemple à l'état de solution limpide stérile,
- pour des préparations injectables
- ou encore comme colle biologique
ainsi que sous d'autres formes détaillées plus loin.

Il peut être utilisé dans des compositions en association
- à pH réduit, à titre de véhicule pour d'autres principes actifs pour libération instantanée de ces derniers. Ces autres principes actifs sont de manière avantageuse choisis dans le groupe constitué par les facteurs de la coagulation, par exemple la thrombine, les agents anti-infectieux, par exemple le métronidazole, les antibiotiques notamment les macrolides tels que la gentamicine, les fluoroquinolones tels que la péfloxacine, les anti-inflammatoires, par exemple les anti-inflammatoires stéroïdiens ou non stéroïdiens acylcarboxyliques ou dérivés oxicam, les antimycotiques, les facteurs de croissance notamment les facteurs de croissance osseux tels que la somatotropine et les facteurs de croissance épidermiques tels que EGF.

Toutefois dans certaines utilisations spécifiques, par exemple, en ophtalmologie ou dans le cas d'associations médicamenteuses incorporant des principes actifs incompatibles tels que l'antibiotique glycopeptidique vancomycine, avec un pH acide pour ne citer que ces deux exemples, il est d'évidence préférable de disposer d'un collagène à pH neutre.

Il peut être également avantageux de disposer de collagène à pH neutre à titre de véhicule pour une libération prolongée de principes actifs tels que par exemple les facteurs de la coagulation ou encore dans le cas où on cherche à associer à l'activité de ces derniers une action mécanique. Aussi il est également particulièrement avantageux d'utiliser le collagène conforme à la présente invention.
- ou en combinant dans des formes solides stratifiées les deux associations précédentes à pH acide et à pH neutre dans des applications particulières qui vont être décrites ultérieurement.

A titre d'exemples des très nombreuses présentations du collagène selon l'invention on peut citer :
- formes liquides : gel, notamment gel injectable, goutte, mousse, spray,
- formes pseudo-solides : films, feuilles, membranes, bandeaux ou plaques ( « patch »)...
- formes solides : poudres, éponges, laminés / stratifiés, films, membranes, fils, sutures...

On peut également mettre à profit les caractéristiques d'acidité et de solubilité du collagène stérile conforme à l'invention dans des compositions pharmaceutiques ou cosmétiques en particulier dans des compositions hydratantes.

On peut utiliser, par exemple, le collagène à pH réduit et sous forme pseudo-solide obtenu conformément au procédé de l'invention, seul ou associé à d'autres principes actifs tels que la vitamine E, la vitamine C, en particulier à titre d'hydratant tissulaire au niveau épidermique et de véhicule de principe(s) actif(s) dans un masque pour visage.

Ces présentations ainsi que quelques autres vont être illustrées dans la suite de la présente description.

Dans le cadre de la présente invention, on entend par
« collagène natif » : un collagène qui n'a subi aucune modification dans sa structure initiale, l'intégrité de ses chaînes polypeptidiques étant préservée (y compris les télopeptides) et la structure hélicoïdale de la molécule étant demeurée intacte.
«collagène atélopeptidique » : un collagène ayant subi une protéolyse limitée par digestion enzymatique à l'aide d'une enzyme protéolytique autre que la collagénase telle par exemple la trypsine, la papaïne...ou mieux encore la pepsine conduisant à l'enlèvement de ses télopeptides. Bien entendu, s'il était à l'état natif il conserve sa structure trihélicoïdale.
« collagène stérile » : un collagène stérile au sens des normes de l'Office Mondiale de la Santé, Pharmacopée Européenne et USA (USP 22), c'est à dire certifié stérile après analyse microbiologique selon les critères reconnus par les administrations concernées.
« collagène liquide » : un collagène en solution, sous forme de suspension, de dispersion ou de gel.
« collagène pseudo-solide » : un collagène sous forme de gel ne s'écoulant pas, formant une masse compacte et homogène d'humidité supérieure à 75 %.
« collagène solide » : un collagène ne renfermant pas plus de 10 % à 25 % d'humidité, pouvant se présenter sous une forme solide, par exemple, de poudre, film, éponge, membrane etc...
« collagène natif à pouvoir polymérisant » : un collagène natif préparé selon une variante du procédé conforme à la présente invention présentant, à l'état liquide ou une fois solubilisé à partir d'une forme solide, un caractère fluide à une température supérieure à 37 °C. Il se transforme en une masse homogène et se présente sous une forme « pseudo-solide » à une température inférieure ou égale à environ 37 °C. Un collagène atélopeptidique peut également présenter ledit pouvoir polymérisant.
« extrait solubilisé et purifié » : un extrait collagénique, non stérile, à pH réduit (de 1,5 à 6,5, habituellement de 3 à 6, mieux encore de 4 à 5), dont le taux de cendres sulfuriques est en général inférieur à 2 % et le taux de lipides est en général inférieur à 1 % ; et comprend de l'hydroxyproline de 12 % à 13,9 %, azote total de 17 % à 18,7 %, ne contenant aucune trace de tryptophane ni de chaîne polypeptidique inférieure à 95000 Da.

Un tel extrait peut être obtenu à partir de peaux fraîches chaulées, sèchées, telles que des peaux de veaux, de lapins et / ou de tissus conjonctifs notamment de bovins, caprins, porcs, ovins, chevaux, lapins, cerfs, autruches, poissons etc... ou à partir d'os, de cornées ou encore de tendons animaux frais ou séchés ( par exemple tendons d'Achille d'autruches), ou encore à partir de fibres ou de poudres purifiées de collagène. Il peut aussi être extrait de placenta d'origine humaine.

L'extrait utile dans le cadre de la présente invention est préparé par une série de traitements comprenant en résumé des étapes de :
chaulage / épilation / lavages / déchaulage / élimination de l'épiderme et des tissus sous-cutanés / découpage / essorages / immersion dans NaOH 1 N pendant 12 h à 25 °C/ lavages pour élimination totale de NaOH / broyage / découpe en films / délipidation dans un bain de Triton X100 à 0,01 % / élimination éventuelle des aminoglycanes bain de K₂HPO₄ / lavages / essorages / lavage à l'eau purifiée / essorages / acidification dans acide acétique, acide monochloroacétique ou acide citrique 10⁻² M / dilution éventuelle de l'extrait de collagène.

Le procédé de la présente invention peut s'appliquer à différents collagènes de Type I, II, III, IV etc... Toutefois dans ce qui suit, et à titre purement illustratif et non limitatif les expressions « extrait de collagène », « collagène » se rapportent, sauf indication contraire, à un extrait de collagène ou au collagène majoritairement de type I.

Ainsi dans le cadre de la présente invention l'extrait collagénique est constitué majoritairement de collagène de type I, à savoir renfermant plus de 90 % de collagène de type I. L'extrait peut aussi être constitué en totalité de collagène de type I, par exemple en poursuivant l'élimination de collagène de type III suivant des techniques connues de dialyse, centrifugation et précipitation différentielle au chlorure de sodium.

Le collagène de type I est un polymère de haut poids moléculaire de 285000 Daltons constitué par l'association en triple hélice de deux chaînes polypeptidiques identiques appelées α₁(I)₂ et d'une chaîne polypeptidique appelée α₂(I)₁. Il appartient à la famille des collagènes fibrillaires (type I, II, III et V).

Le collagène présente un pic de dénaturation qui dépend de son origine, de la méthode d'extraction y conduisant et de son état réticulé ou non. A l'état non réticulé, le pic de dénaturation se situe à 35 - 45 °C.

Le collagène majoritairement de type I, a un rapport α₂(I)₁/α₁(I)₂ de 0,48 à 0,52 comme mesuré par densitométrie effectuée sur gel de polyacrylamide après migration des chaînes par électrophorèse.

L'inactivation virale de cet extrait et de la pepsine éventuellement présente peut être effectuée par traitement chimique par passage en alternance dans des solutions tampons à pH supérieur ou égal à 13 et à pH inférieur ou égal à 2,5. On effectue en général des traitements connus en soi, tels qu'un trempage dans un bain d'hydroxyde de calcium à 4 % associé à du sulfure de sodium à 3 % suivi d'un deuxième bain de métabisulfite de sodium à 0,5 % associé à du chlorure d'ammonium à 2 %.

La neutralisation éventuelle de la pepsine est réalisée par un traitement sodique ramenant le pH à 6,8 - 7,4, comme exposé par exemple dans le document FR 2 586 703.

De même l'élimination d'éventuels agents responsables des encéphalopathies spongiformes bovines appelés « prions » peut être réalisée également de manière connue, comme décrit dans les directives de l'OMS (WHO/COS/VHP/92.104) et de la Communauté Européenne du 11 décembre 1991, par exemple. Cette élimination peut ainsi consister en un traitement avec une solution d'hydroxyde de sodium 1 N pendant 1 h à 48 h à 25 °C ou encore en un traitement à l'hypochlorite de sodium pendant au moins 1 h à 25 °C.

Selon un premier aspect, la présente invention a donc plus précisément pour objet un procédé de préparation d'un collagène à partir d'un extrait solubilisé et purifié, éventuellement pepsiné, de collagène natif non stérile. Habituellement les étapes consistent à mélanger et cisailler ledit extrait dans un mélangeur à double couteaux transversaux pendant une durée de 1 min à 60 min, avantageusement de 15 à 40 min et de préférence de 10 à 20 min à une vitesse d'agitation de 100 tours par min à 10000 tours par min, en général de 500 tours à 7000 tours par min et de préférence de 1000 tours à 5000 tours par min, tout en contrôlant la température, puis à stériliser ledit extrait.

De manière avantageuse selon une première variante d'exécution et lorsque l'extrait n'est habituellement pas pepsiné - ci-après « variante 1 » désignera pour simplifier, le mode de réalisation du procédé de l'invention dans lequel l'extrait n'est pas pepsiné sauf indication expesse du contraire -, la température, qui est toujours contrôlée, ne dépasse pas en général, 40-50 °C, voire 60-80 °C en présence d'agent réticulant -selon un autre mode de réalisation alternatif de cette variante décrit plus en détails plus loin -.

De préférence, selon la première variante du procédé de la présente invention, l'extrait n'étant pas pepsiné, on augmente de manière progressive sa température de l'ambiante jusqu'à atteindre 40 °C à 50 °C dans ledit mélangeur, tout en élevant par paliers la vitesse d'agitation initiale de 500 tours par min pour atteindre jusqu'à 5000-7000 tours par min, et à chaque phase d'augmentation de la viscosité, on procède à une dilution de l'extrait On maintient ainsi le collagène a une viscosité élevée choisie tout en diminuant sa concentration. Avantageusement, ladite viscosité élevée est de 15000-20000 cps (15Pa.s-20Pa.s). Par exemple, lorsque ladite température maximale est de 42-44°C, on laisse reposer ledit extrait avant ladite étape de stérilisation.

On observe, après stérilisation, que le collagène ainsi préparé à partir d'un extrait non pepsiné a conservé son état natif et a acquis des propriétés améliorées notamment d'élasticité et de résistance mécanique. Il présente un pouvoir adhésif et polymérisant.

De manière préférée, ledit extrait dans ledit mélangeur est porté de la température ambiante à une température d'au plus 40-50 °C, par paliers de 2°C à 10°C, de préférence 3°C à 5 °C, tout en augmentant simultanément la vitesse d'agitation, aussi par paliers de 500 à 1000 tours par min, pour atteindre une vitesse de 1500 tours par min à 7000 tours par min, de préférence 5000 tours par min.

Il est en effet important d'élever la température par paliers car une augmentation brutale de la température dénature de manière temporaire le collagène sans accroître suffisamment ses propriétés élastiques. Cette température est portée à au moins 40 °C, car en dessous de cette température la filtration ultérieure est rendue mal aisée voire impossible.

Il est également important d'augmenter la vitesse d'agitation par paliers pour mieux contrôler la viscosité choisie élevée tout en diminuant la concentration du collagène, comme déjà mentionné.

La concentration de collagène dans l'extrait est de 0,001 % à 15 %, en général de 0,1 à 10 %, et de préférence de 3 à 6 %.

Comme décrit précédemment, la température dans ledit mélangeur peut toutefois atteindre des valeurs supérieures pouvant aller jusqu'à 80°C en présence d'agents réticulant tels que le glutaraldéhyde et les azyl acides tels que l'hydrazine ou tout autre agent réticulant compatible avec le collagène. Lorsqu'ils sont présents, ces agents ont une concentration finale dans l'extrait de 0,00075 % à 0,1 %, de préférence 0,0075 % à 0,01 %, dans le cas du glutaraldéhyde et de 0,1 % à 2 %, de préférence 0,5 % à 1,5 %, pour l'hydrazine. Le collagène ainsi «réticulé» possède en conséquence une température de prise en masse plus élevée pouvant se situer aux environ de 40-50 °C et présente un temps de résorption ***in vivo*** supérieur pouvant aller jusqu'à 8 semaines à 12 semaines comparé au collagène non réticulé.

Selon un mode de réalisation particulier de cette variante du procédé selon l'invention, consistant à obtenir le collagène natif stérile sous la forme d'un liquide visqueux, on procède comme suit :
- on prépare un collagène natif non stérile, extrait de peaux de lapins ou de tendons d'Achille d'autruche, à une concentration suffisamment élevée de l'ordre de 10 à 15 %.
- l'extrait de collagène natif non stérile est ensuite soumis à une agitation et à un cisaillement dans le mélangeur à double couteaux transversaux déjà mentionné, à vitesse modérée de 500-1000 tours par min et à température ambiante, de l'ordre de 20 °C.
- on augmente par pallier la vitesse de rotation et de cisaillement ce qui a pour effet d'augmenter fortement la viscosité de l'extrait. Cette viscosité peut prendre des valeurs de 7000 cps à 20000 cps. A partir de 15000 cps, l'extrait présente un aspect quasi élastique, rendant extrêmement difficile son agitation et son cisaillement dans le mélangeur.
- à chaque phase d'augmentation de la viscosité, on procède à une dilution de l'extrait par addition d'eau pour préparation injectable (PPI) ou d'acide citrique 10⁻² M et on augmente la vitesse d'agitation et la température dans le mélangeur qui sont ainsi élevées par paliers jusqu'à l'obtention d'une viscosité très élevée de l'ordre de 20000 cps (20Pa.s).
- on répète cette opération à plusieurs reprises, de préférence quatre à cinq fois jusqu'à atteindre une température de 35 °C, une vitesse de 3000 à 5000 tours par min et une concentration de collagène de l'ordre de 3 à 6 %.
- on maintient le produit à cette température de 35 °C et à cette vitesse de 3000 à 5000 tours par min pendant quelques min, de préférence 3 à 5 min.
- on augmente la température dans le mélangeur jusqu'à atteindre 40-50 °C, de préférence 40-45 °C, dans le cas du collagène non réticulé. A cette température, on observe de manière tout à fait surprenante une liquéfaction de l'extrait avec une chute de la viscosité à environ 40-100 cps.
- on filtre immédiatement l'extrait collagénique, préparé comme il vient d'être exposé ci-dessus, en profondeur et à une température opératoire contrôlée de 40-50 °C sur membranes de porosité 0,45 µ-0,22 µ.
- on procède ensuite à sa stérilisation qui est réalisée de manière préférée par une filtration absolue sur membrane de porosité 0,22 µ, telle qu'une membrane Millidisck® commercialisée par la société Millipore, à la température opératoire contrôlée de 40-50 °C. Le temps de filtration à la température de 40-50 °C est choisi de manière à ne pas dépasser 1 h, mieux encore 10 à 20 min. On peut aussi procéder à la stérilisation, d'une manière également préférée, par ajout d'acide peracétique, produit en particulier par la société Air Liquide sous la dénomination Soproper®, à la concentration finale dans l'extrait filtré en profondeur de 0,5 % à 50 %, et de préférence de 5 % à 15 % en poids par rapport au poids sec du collagène. La neutralisation de cet agent stérilisant est réalisée par addition de thiosulfate de sodium à raison de 2 à 20 g pour 1 g d'acide peracétique et de préférence de 4 g à 12 g/g d'acide peracétique. Le temps de contact nécessaire à la stérilisation par l'acide peracétique est de 1 h à 24 h à température ambiante, de préférence de 2 h à 4 h.

Selon un autre mode de réalisation particulier de cette première variante du procédé conforme à la présente invention, consistant à obtenir le collagène natif sous la forme d'un gel pseudo-solide, la concentration de collagène étant de 0,5 % à 10 %, il est aussi avantageux de le stériliser directement par irradiation à la dose de 2,5 Mrad (2,5.10⁻⁵ Gy) (rayonnements ionisants bêta ou gamma). On observe de manière surprenante que le gel pseudo-solide demeure compact et homogène et qu'il ne se produit alors aucune séparation en deux phases contrairement aux gels classiques, et conserve ses propriétés physico-chimiques et son pouvoir polymérisant. Ce gel pseudo-solide stérilisé par cette méthode peut de manière avantageuse être liquifié pour être associé à des principes actifs stériles sensibles à l'irradiation ionisante, tels que des facteurs de la coagulation, des antibiotiques, des antimycotiques, etc. pour l'obtention de préparations liquides ou solides, notamment les préparations citées à titre d'exemples ci-après : éponges, poudres, films membranes, "patch"...

Lorsqu'il est utilisé ce mode de stérilisation sur des préparations solides de collagène obtenu selon cette première variante du procédé, seul ou associé à des principes actifs résistants aux rayonnements ionisants choisis dans les classes thérapeutiques citées dans le présent document, il ne se produit aucune modifications des propriétés physico-chimiques et du pouvoir polymérisant et adhésif de cette forme de collagène.

Selon cette variante 1, le procédé selon l'invention peut comprendre en outre une étape de neutralisation. La neutralisation peut être réalisée avant ou après l'étape de stérilisation. Cette neutralisation peut être réalisée avec l'hydroxyde de sodium, par exemple, afin d'ajuster le pH à 6,8 - 8,2 et de préférence à 7,0 - 7,4. On constate de manière tout à fait surprenante que cette neutralisation du collagène à l'état de liquide visqueux et à la température de 40-50 °C ne provoque pas de précipitation du collagène. L'extrait reste à l'état de liquide visqueux limpide ou translucide et se prend rapidement en masse dans les mêmes conditions que le collagène à pH réduit. C'est très avantageux car la réalisation de certaines préparations comme un gel à base de collagène, ou encore une association de collagène avec la vancomycine, était empêchée jusqu'ici en raison de la précipitation du collagène à pH neutre.

Les analyses de la molécule de collagène de l'invention par les techniques classiques déjà mentionnées confirment l'intégrité des structures primaire, secondaire et tertiaire du collagène ainsi neutralisé.

### Caractéristiques du collagène obtenu selon la variante 1 :

On constate de manière tout à fait surprenante que le collagène, stérilisé conformément à la première variante du procédé de la présente invention, a conservé son état natif.

A la température de 40-50 °C, c'est un liquide translucide visqueux.

Sa viscosité varie en général de 20 cps à 300 cps (20.10⁻³ à 300.10⁻³Pa.s) et de préférence de 30 à 200 cps (30.10⁻³ à 200.10⁻³Pas) et mieux encore de 40 à 100 cps (40.10⁻³ à 100.10⁻³Pa.s).

Son pH est en général de 1,5 à 6,5, de préférence de 2 à 5 et mieux encore de 3 à 4.

Sa concentration est en général de 0,001 à 15 %, de préférence de 0,1 à 10 %, mieux encore de 3 à 6 %.

Il se prend en masse très rapidement à une température inférieure ou égale à 38 °C. Il prend une forme pseudo-solide présentant des propriétés adhésives remarquables de colle hémostatique. Son temps de prise en masse à une température d'environ 38 °C se situe à moins d'1 min à une concentration supérieure ou égale à 5 %, entre 1 min et 2 min à une concentration de 3 % à 4 % et à environ 3 min à une concentration inférieure à 2 %.

Le collagène stérile natif obtenu présente en outre les autres caractéristiques ou propriétés importantes suivantes :
- collagène majoritairement, à savoir à 90 % ou plus, de type I soit un rapport α₂(I)₁/α₁(I)₂ étant de 0,48 à 0,52
- taux d'humidité : supérieur à 80 %
- aspect : liquide à une température supérieure à 38 °C environ et collant au toucher ; masse gélifiée homogène et compacte pseudo-solide à une température inférieure à 38 °C environ.
- Azote total : 17,0 % à 18,7 %
- Hydroxyproline : de 12 % à 13,9 %
- dépourvu de tryptophane, d'aminoglycanes et de polypeptides de poids moléculaire < 95000 Dalton
- lipides < 1 %
- cendres sulfuriques < 2 %
- pic de dénaturation : 38 °C à 45 °C tel que mesuré par thermographie différentielle (DSC).
- pouvoir adhésif
- pouvoir polymérisant
- conservation à température ambiante
- immédiatement prêt à l'emploi.

Aussi la présente invention a également pour objet un tel collagène présentant les caractéristiques précitées.

Préparations à base de collagène obtenu selon cette première variante du procédé conforme à la présente invention et applications :

Comme déjà mentionné le collagène conforme à l'invention à pH acide ou neutre possède un pouvoir de polymérisation et des propriétés adhésives remarquables. Ces dernières associées à ses propriétés hémostatiques et de résorption lui confèrent la qualité d'une colle hémostatique résorbable.

Le collagène de l'invention peut être réparti de préférence dans des seringues ou flacons de 2 à 5 ml en verre de préférence et qui sont maintenus à une température permettant sa polymérisation ou prise en masse entre 0 °C et 38 °C, mieux encore à température ambiante.

Une autre forme avantageuse de cette colle hémostatique résorbable conforme à l'invention est la forme sèche, en poudre ou éponge. Cette forme ne nécessite pas de préparation préalable avant son emploi.

Pour obtenir une telle forme on soumet le collagène de l'invention, obtenu à l'état visqueux, par exemple, à une lyophilisation.

Le collagène à l'état liquide de l'invention est réparti uniformément dans des récipients (plateaux de lyophilisation) dans des conditions aseptiques conduisant à des lyophilisats d'une épaisseur de 1 à 20 mm, de préférence de 2 à 10 mm, mieux encore de 3 à 5 mm. Le lyophilisât obtenu est de couleur crème et d'aspect filmogène, légèrement élastique. Son humidité est de 1 % à 25 % et de préférence de 5 % à 15 %. Les lyophilisats peuvent subir un broyage pour donner une poudre de faible granulométrie choisie.

Pour les préparations de poudre il est préférable d'avoir une concentration de collagène avant lyophilisation de l'ordre de 3 à 6 %, ou plus, pour assurer une meilleure pulvérisation.

La poudre après broyage peut être répartie dans des flacons stériles en polyéthylène à double bouchon ou un autre récipient permettant le maintien de la stérilité et facilitant la pulvérisation sur la surface hémorragique.

Dans le cas des éponges, la concentration de collagène doit être relativement faible de préférence de 1 à 2 %.

Les éponges filmogènes peuvent être conditionnées individuellement, après lyophilisation, en doubles sachets ou en plaquettes blisters avec fermeture thermosoudée. Elles sont par exemple de formes carré, triangulaire ou circulaire et présente un côté ou un diamètre de 1 cm à 30 cm selon les applications.

L'action des deux formes sèches mentionnées ci-dessus est semblable à celle de la forme liquide déjà décrite. Toutefois l'éponge filmogène exerce en outre une action mécanique souvent indispensable en cas d'hémorragie et de saignement en nappe, en particulier abdominal.

A cette colle quelle que soit sa forme, notamment liquide visqueux, pseudo-solide, éponge filmogène ou poudre, on peut associer des principes actifs notamment pharmaceutiques comme par exemple des facteurs de coagulation intervenant dans l'accélération de l'hémostase (tels que, par exemple, la thrombine, le fibrinogène, le facteur XII etc...), ou encore des antibiotiques, notamment dans le cas de la chirurgie osseuse.

Lorsqu'elle renferme des facteurs de la coagulation, on peut avantageusement associer à cette colle des agents stabilisant, tels que par exemple des polyols (comme le glycérol, un polyalkylèneglycol dont le radical alkylène présente de 1 à 4 atomes de carbone, tel que le polyéthylèneglycol, le polypropylèneglycol, notamment) ou des mono-, di- ou polysaccharides (tels que le glucose ou le saccharose).

Comme autres préparations obtenues à partir de la forme liquide visqueuse de collagène selon la variante 1 du procédé de l'invention on peut citer :
Un «patch» pseudo-solide, à titre de véhicule de principes actifs à usage local extra-épidermique, ou encore un spray cicatrisant indiqué notamment chez les Grands Brûlés ainsi qu'un masque hydratant à usage cosmétique.
Le «patch» peut incorporer un ou des antibiotiques, anti-inflammatoires, antiseptiques, antibactériens, antifongiques, antimycotiques ainsi que d'autres substances physiologiquement et pharmaceutiquement actives, notamment l'acide salicylique ou la nicotine. On réalise l'addition de ces principes actifs à une température supérieure à 40 °C jusqu'à homogénéisation. Le mélange est dégazéifié et propulsé par un système sous pression, par exemple, tel qu'une pompe doseuse et est distribué dans des conditionnements étanches (Blisters ou films de polyéthylène ou similaires, par exemple). Après refroidissement du liquide à température ambiante, on procède à une fermeture par thermosoudure des conditionnements.

Les concentrations de collagène utilisées dans le «patch» varient de 1 à 8 % et de préférence de 2 à 5 %.

Lorsqu'ils sont présents les antibiotiques ont les concentrations suivantes en mg d'antibiotique / mg de collagène.

Gentamicine : de 0,01 à 2 mg/mg, mieux encore de 0,1 à 0,7 mg/mg et de préférence de 0,15 mg/mg à 0,5 mg/mg.

Vancomycine : de 0,01 à 2 mg/mg, mieux encore de 0,1 à 0,7 mg/mg et de préférence de 0,15 mg/mg à 0,5 mg/mg.

Péfloxacine : de 0,01 à 2 mg/mg, mieux encore de 0,05 à 0,5 mg/mg et de préférence de 0,075 mg/mg à 0,25 mg/mg.

Les épaisseurs du «patch» peuvent varier de 1 à 5 mm ou plus. On préfère toutefois des «patch» d'épaisseur 1,5 à 2,5 mm.

Selon l'utilisation envisagée, la longueur et la largeur sont adaptées.

Un «patch» obtenu conformément à la présente invention possède une forme pseudo-solide, est souple, résistant, fortement hydraté (teneur en eau supérieure à 80 %) a un aspect translucide.

Il s'applique aisément sur les surfaces tissulaires internes ou externes qu'elles soient endommagées ou saines. Dans le cas des applications épidermiques externes, le «patch» peut être fixé sur un adhésif hypoallergénique imperméable permettant son adhésion et son maintien sur la surface tissulaire.

Le masque est obtenu en faisant couler le collagène seul ou associé à des principes actifs tels que par exemple, des vitamines (C, E...), un tensioactif comme le chlorure de benzalkonium, des hydratants comme le glycérol, sur un tapis roulant par exemple à une faible vitesse de défilement permettant d'obtenir un film homogène et d'épaisseur régulière. Après refroidissement, il est découpé selon des dimensions appropriées sous forme pseudo-solide et est conditionné dans des sachets hermétiques (en aluminium avec pellicules internes de polyéthylène, par exemple).

Les concentrations de collagène utiles dans la préparation d'un tel masque sont en général de 0,2 à 3 %, mieux encore de 0,5 à 2 % et de préférence de 1 à 1,5 %.

Les formes et dimensions ne sont aucunement limitées. Un masque pour le visage a habituellement la forme et les dimensions d'une feuille de type A4 (21 x 29,7 cm). Les épaisseurs sont généralement de 0,5 à 3 mm et de préférence de 1 à 1,5 mm.

Le masque présente une forme pseudo-solide, est très souple, doux au toucher, translucide et est fortement hydraté (teneur en eau supérieure à 97 %). Il s'applique très facilement sur la peau. On peut y additionner un ou des colorants et d'autres principes actifs compatibles avec le collagène.

Le spray renferme le collagène et les principes actifs dans les mêmes concentrations ainsi que les mêmes associations que celles décrites pour le «patch». Il est conditionné avantageusement dans des flacons ou pulvérisateurs monodoses de 5 à 25 ml de préférence en verre ou polyéthylène.

Avant emploi, le collagène, à l'état polymérisé à température ambiante, est liquéfié par chauffage au bain marie ou à l'étuve pendant 2 à 3 min dans son conditionnement d'origine. On pulvérise à distance immédiatement avant la prise en masse du collagène sur la surface tissulaire.

Le collagène ainsi pulvérisé adhère à la surface tissulaire et se polymérise très rapidement sur celle-ci formant ainsi un film homogène cicatrisant et hémostatique. Un tel spray est particulièrement indiqué lorsqu'on vise une action cicatrisante et/ ou une libération prolongée de substances actives tels que des antibiotiques, antiseptiques, antimycotiques, anti-inflammatoires etc...).

Ce dispositif de pulvérisation à distance présente l'avantage d'éviter tout contact de l'opérateur avec la surface tissulaire endommagée et donc tout risque d'infection locale éventuelle.

Une des utilisations de ce spray, particulièrement intéressante et rendue possible en raison des caractéristiques particulières du collagène conforme à la présente invention, concerne notamment les grands brûlés pour lesquels une garantie de stérilité et de rapidité des soins est une nécessité.

Comme autres préparations obtenues, à partir de la forme liquide visqueuse de collagène, selon la variante 1 du procédé de l'invention on peut encore citer : les films ou membranes obtenus par coulage sur un tapis en polyéthylène, par exemple, suivi d'un séchage sous air, en étuve ventilée ou sous-vide. La température opératoire est de 10 °C à 35 °C, et de préférence de 20 °C à 25 °C.

Pour obtenir par exemple des films d'une épaisseur inférieure à 1 mm, de préférence de 25 µm à 250 µm, on utilise habituellement des solutions de collagène dont la concentration varie de 2 à 5 %, de préférence de 2,5 à 3,5 %. Les durées de séchage peuvent varier de 1 h à 120 h. Avantageusement les durées de séchage sont de l'ordre de 24 h. En effet on observe que les meilleurs résultats sont obtenus avec un séchage relativement lent et un débit d'air relativement faible.

Les films ou membranes ainsi obtenues ont un taux d'humidité de 1 à 15 % et de préférence de 5 à 10 %. Ils sont découpés dans des conditions aseptiques soit manuellement soit automatiquement et conditionnés dans des emballages étanches et imperméables.

A l'instar des autres formes précitées, les films ou membranes peuvent contenir des principes actifs tels que des antibiotiques, des antiseptiques ou encore des anti-inflammatoires, etc. Ils peuvent être produits à partir de collagène acide (pH réduit) ou neutre.

Ils sont utilisés à usage externe ou interne pour la cicatrisation et / ou la libération d'un principe actif, médicamenteux par exemple. En raison de leur élasticité au contact d'une surface humide hémorragique, ils peuvent avantageusement former une enveloppe qui adhère à la zone du tissu endommagé.

Dans les applications ophtalmologiques ils sont produits à partir d'un collagène à pH de 6,8 à 7,2 de préférence. Dans ce cas, les films ou membranes peuvent prendre la forme d'un cylindre réservoir ou de lentille de contact transparents, pour une délivrance locale prolongée de principes actifs au niveau oculaire, par exemple. Dans le cas où on souhaite utiliser cette présentation de collagène sous une forme liquide (gouttes / collyre), on conserve le collagène dans des capsules ou autres conditionnements monodoses qui doivent être réchauffés avant emploi.

Dans le cas des applications ophtalmologiques, les concentrations de collagène à pH neutre sont pour les membranes ou lentilles de 1 % à 2,5 % et de préférence 0,5 % à 2 %, et pour les gouttes oculaires à pH neutre, la concentration de collagène est de 0,1 % à 1,5 %, de préférence 0,2 % à 0,5 %.

La présente invention a donc aussi pour objet les compositions décrites ci-dessus pour lesquelles on a cité, à titre illustratif seulement, quelques-unes des formes galéniques possibles.

Selon une deuxième variante du procédé selon la présente invention, l'extrait est soumis, avant le traitement mécanique dans ledit mélangeur avec contrôle de la température, à une protéolyse limitée par une enzyme appropriée, à l'exception de la collagénase, telle que par exemple la trypsine, la papaïne, et la pepsine, de préférence la pepsine, comme il va être exposé ci-dessous. En général, la concentration du collagène dans l'extrait est de 0,1 % à 2 %, mieux encore de 0,2 % à 1 % et de manière la plus préférée de 0,3 % à 0,5 %.

On traite alors ledit extrait (solubilisé, purifié) pepsiné comme suit :
- on procède à des filtrations clarifiantes « en cascade » sur membranes de différentes porosité, par exemple 100 - 50 puis 20 µ, 1 µ, jusqu'à 0,45 microns.
- on effectue ensuite une nouvelle étape de purification par précipitations différentielles aux sels de métaux alcalins, par exemple au chlorure de potassium ou de sodium, de préférence au chlorure de sodium, suivie éventuellement de dialyses contre des tampons phosphate K₂HPO₄, Na₂HPO₄ et enfin de centrifugations pour récupérer le collagène précipité.
- on homogénéise le culot ainsi obtenu, la concentration de collagène étant de 1 % à 30 % par agitation douce dans de l'eau purifiée puis on le lave 2 à 3 fois avant remise en suspension dans l'eau purifiée et maintien pendant 48 h sous agitation douce à une température de 0 °C à 20 °C, et, de préférence, une température de 0 °C à 10 °C.
- on procède à une centrifugation ou décantation avec essorage de cette suspension. Le collagène est recueilli dans une solution d'acide citriqué 10⁻² M à pH réduit de 2 à 5, de préférence 2,5 à 3,5 et dont le volume est calculé en fonction de la concentration finale choisie de collagène.
- on laisse reposer le culot dans cette solution acide sans aucune agitation à faible température de 0°C à 10°C pendant 2 h à 48 h, et de préférence 12 h à 24 h. Le culot de collagène gonfle, perd sa coloration blanche pour devenir translucide.
- on transfère le culot dans un mélangeur à double couteaux transversaux, comme par exemple, le cutter - mélangeur DITO-SAMA déjà mentionné, puis on augmente progressivement l'agitation et le cisaillement sans dépasser 10000 tours par min pour atteindre une agitation, de préférence, de 1000 à 5000 tours par min, mieux encore de 2000 à 3000 tours par min, de manière encore plus préférée 2500 tours par min, tout en contrôlant l'augmentation de température par paliers de 2°C à 10°C, de préférence de 3°C à 5°C, de manière à ne pas dépasser 35 °C, de préférence 25 °C, jusqu'à solubilisation totale. La solution obtenue est limpide.

Ici encore le collagène est préparé à partir d'un extrait de collagène, de préférence, majoritairement de type I.

Selon cette Variante 2, la solution de collagène atélopeptidique ainsi préparé et purifié peut subir alors de manière avantageuse une stérilisation par filtration absolue sur membrane de porosité 0,22 µ à la température opératoire, à savoir selon cette variante de préférence à une température de 20-25°C, ou encore par ajout d'acide peracétique dans des conditions identiques à celles déjà décrites précédemment, ou encore par rayonnement ionisants (2,5 Mrads - 2,5.10⁻⁵Gy) sur une forme préalablement séchée, éventuellement destinée à être solubilisée en milieu liquide stérile pour être associée à une préparation stérile de principes actifs sensibles aux rayonnements ionisants choisis dans les classes thérapeutiques désignés dans le présent document

La neutralisation éventuelle est effectuée, de préférence, après l'étape de stérilisation à température ambiante. A température ambiante, on observe qu'un précipité commence à se former dès la deuxième heure de neutralisation. Aussi il est particulièrement avantageux de conserver le collagène à une température de 0°C à 8°C et d'effectuer les associations ou mélanges du collagène avec les autres principes actifs, incompatibles à pH réduit (de 1 à 6,5), au cours des deux premières heures après neutralisation, de préférence dans la première heure, et mieux encore dans les 30 min, et de commencer alors à procéder, notamment aux éventuelles opérations de congélation, lyophilisation, séchage sous air ou sous vide.

Caractéristiques du collagène obtenu selon la variante 2 :

Le collagène atélopetidique ainsi préparé est stérile et a conservé sa structure trihélicoïdale. Il se présente sous la forme d'une solution limpide et de faible viscosité de 2 cps à 40 cps (2.10⁻³ à 40.10⁻³Pa.s), de pH acide ou neutre si besoin.

Les autres caractéristiques du collagène ainsi produit sont les suivantes :
- collagène à 90 %, ou plus, de type I à savoir un rapport α₂(I)₁/α₁(I)₂ de 0,48 à 0,52
- pic de dénaturation : 38 °C à 45 °C tel que mesuré par thermographie différentielle (DSC).
- Azote total : 17,0 % à 18,7 %
- Hydroxyproline : de 12 % à 13,9 %
- dépourvu de tryptophane, d'aminoglycanes et de polypeptides de poids moléculaire < 95000 Dalton
- lipides < 1 %
- cendres sulfuriques < 2 %

Aussi la présente invention a encore pour objet un tel collagène natif stérile atélopeptidique.

Préparations à base de collagène obtenu selon la variante 2 et applications. A titre d'exemples on peut citer :
- utilisation telle quelle sous forme liquide à pH acide, en tant qu'hydratant tissulaire ou associée dans des préparations à des principes actifs cosmétiques par exemple, à des concentrations de 0,1 % à 3 % ; en association dans des préparations thérapeutiques cicatrisantes, par exemple en présence de principes actifs en dermatologie tels que des anti-inflammatoires, des antiseptiques locaux, des antibactériens, des antifongiques, l'acide salicylique...Ces formulations se présentent sous la forme de crème, gel, mousse ou pommade à usage externe, le collagène y étant incorporé à des concentrations de 1 % à 3 %. Elles peuvent aussi se présenter sous la forme d'un liquide ou d'un spray à application externe, la concentration de collagène étant alors de 0,1 % à 1 %. Les principes actifs mentionnés sont utilisés aux doses thérapeutiques connues.
- utilisation à pH neutre sous forme de pâte ou de gel en association avec des phosphates et des sels alcalins (chlorure de sodium, par exemple), la concentration de collagène étant alors de 2 % à 7 %, par exemple, dans la correction de dépressions cutanées, telles que rides et ridules, ou encore pour le renforcement des muscles lisses, comme dans le cas d'incontinence urinaire ou enfin pour la fabrication de substituts tissulaires, tels que valves cardiaques, par exemple, ou de substituts osseux le collagène étant alors associé, notamment à l'hydroxyapatite ou à du corail.
- utilisation à pH neutre ou acide dans la préparation d'une mousse, à faible concentration de collagène, particulièrement utile en chirurgie et microchirurgie pour la réparation de tissu endommagé et pour assurer le maintien d'espace inter-tissulaire et parenchymateux et éviter des adhérences anatomo-pathologiques entre des tissus endommagés ou lésés - de préférence en chirurgie parenchymateuse, cardio-vasculaire, thoracique, plastique, ou ORL). Cette mousse peut incorporer des principes actifs, en particulier des antibiotiques, antiseptiques et des anti-inflammatoires, dans les mêmes concentrations que celles mentionnées ci-dessus pour les préparations selon la variante 1. Une telle mousse va être décrite plus en détails ultérieurement.

A partir de ce collagène stérile obtenu selon la variante 2, on peut aussi préparer, en particulier à l'aide d'un séchage par lyophilisation, des éponges hémostatiques résorbales particulièrement avantageuses par leur action hémostatique et pour véhiculer localement un ou des principes actifs tels que notamment des agents anti-infectieux et / ou des facteurs de la coagulation.

De telles éponges obtenues à partir de collagène à pH acide selon la variante 2 sont très avantageuses car instantanément solubles une fois mises en contact avec un milieu liquide, permettant la libération immédiate d'un ou de plusieurs principes actifs. Les mêmes éponges obtenues à partir d'un collagène à pH neutre selon la variante 2, dont l'aspect est microfibrillaire, permettent une libération prolongée et exercent de plus une action mécanique avantageuse sur la surface hémorragique à laquelle elles adhèrent.

Dans la préparation de telles éponges on peut utiliser un collagène à pH acide ou neutre à une concentration de 0,1 % à 3 %, de préférence de 0,2 % à 2 % et mieux encore de 0,5 % à 1 %.

Lorsqu'ils sont présents, les antibiotiques suivants gentamicine, péfloxacine et vancomycine sont utilisés aux concentrations déjà mentionnées pour les préparations de la variante 1.

D'autres principes actifs peuvent être également présents, tels que la thrombine à une concentration, en Unités Internationales par mg de collagène, de 0,1 UI/mg à 20 UI/mg, de préférence de 0,5 UI/mg à 10 UI/mg et mieux encore de 1 UI/ mg à 5 UI/mg ou encore le fibrinogène (en mg de fibrinogène par mg de collagène), à raison de 0,1 mg/mg à 20 mg/mg, de préférence de 0,1 mg à 10 mg/mg et mieux encore de 0,5 à 5 mg/mg. On peut y associer en outre d'autres actifs tels que d'autres dérivés sanguins notamment la prothrombine, le facteur Xa, le facteur IXa, le facteur XIII et tout autre activateur ou pro-activateur de mécanismes intrinsèques et extrinsèques de la coagulation associés éventuellement à d'autres substances physiologiques et pharmacologiquement actives telles que des activateurs et inhibiteurs de plasminogène, des antifibrinolytiques, par exemple. D'autres substances peuvent y être également associées par exemple des enzymes spécifiques qui régulent la résorption du collagène, ou encore des agents réticulant.

Les mélanges de collagène selon l'invention et du ou des principes actifs précités sont réalisés dans des récipients en acier inoxydable, en verre ou en polyéthylène et à des températures de 5 °C à 35 °C, de préférence de 10 °C à 30 °C, mieux encore de 15 °C à 20 °C.

Comme déjà mentionné, lorsqu'il est à pH neutre, le collagène doit être utilisé dans les 2 h ayant suivi sa neutralisation. On répartit ensuite le mélange dans les récipients de lyophilisation qui permettent l'obtention de couches d'une épaisseur de 1 à 20 mm, de préférence de 3 à 15 mm et mieux encore de 5 à 8 mm. Ces préparations peuvent ainsi être lyophilisées en monocouche selon les paramètres opératoires suivants : température de congélation de -5°C à -60°C, température de résorption de 20°C à 50°C, en général de 25°C à 40°C et de préférence de 30°C à 35°C.

Les préparations d'épongés ainsi lyophilisées sont de couleur blanche, d'aspect cotonneux, tissé, homogène. Leur humidité est de 1 % à 25 %.

Les éponges conformes à l'invention peuvent être utilisées telles qu'elles ou subir un laminage ***in situ*** (dans la chambre du lyophilisateur) en y exerçant une pression individuelle ou, à l'extérieur dans des conditions aseptiques, par passage sur un laminoir. Cette opération permet d'augmenter la résistance de ce type d'éponges.

On peut avantageusement lyophiliser lesdites préparations en une ou plusieurs couches. Dans ce cas l'adhésion entre les couches se fait de manière préférée au cours de la phase de congélation à des températures choisies de -11°C à -20°C, de préférence de -11°C à -13°C. On obtient alors un stratifié dont les deux couches inférieures, par exemple, peuvent renfermer du collagène à pH acide associé par exemple à la thrombine et au fibrinogène (permettant la libération de ces produits et le formation d'une colle biologique) et dont la couche supérieure est constituée, par exemple de collagène à pH neutre (permettant d'exercer l'action mécanique au niveau de la surface hémorragique comme déjà mentionné). La forme précitée permet de manière très avantageuse de véhiculer sans aucune préparation préalable une colle biologique contrairement aux colles connues. De manière avantageuse, la couche à pH neutre renferme des antibiotiques ce qui autorise leur libération prolongée, particulièrement avantageuse dans le cas des infections osseuses. Le choix de l'ordre et du nombre de couches est indifférent, il est à la portée de l'homme du métier qui adaptera ces derniers selon les applications envisagées.

Dans ces associations conformes à la présente invention, on peut encore inclure des agents stabilisant tels que des polyols, polyalkylèneglycols et polysaccharides ou encore des acides aminés ou autres actifs compatibles avec le collagène, tels que des anti-inflammatoires par exemple, ou encore des agents réticulant comme déjà mentionnés pour augmenter encore de manière avantageuse leur temps de résorption. On peut aussi y associer d'autres agents à action mécanique résorbables permettant d'effectuer des sutures tels que des tissus de cellulose ou de cellulose oxydée ou vicryl® (commercialisé par la société Ethnor).

Par ailleurs, le collagène obtenu toujours selon la variante 2 du procédé de l'invention, à pH acide ou neutre, associé ou non à d'autres principes actifs, peut conduire à une poudre hémostatique après lyophilisation ou séchage. Le collagène mis en oeuvre a alors une concentration de 1 % à 3 %.

La présente invention a donc aussi pour objet les compositions décrites ci-dessus et dont on a illustré quelques-unes des présentations galéniques possibles, représentant des préparations de collagène non polymérisé obtenu selon la variante 2.

On va donner dans ce qui suit des exemples non limitatifs de certains des objets de la présente invention et en référence aux dessins sur lesquels :
- les figures 1 et 2 sont des diagrammes d'électrophorèse sur gel de polyacrylamide montrant la séparation des chaînes polypeptidiques du collagène de type I, respectivement avant et après le traitement mécanique et thermique de l'invention (variante 1).
- les figures 3 et 4 sont des diagrammes de dénaturation thermique du collagène mettant en évidence le caractère natif (non dénaturé) du collagène, respectivement avant le traitement mécanique et thermique de l'invention (variante 1) et après celui-ci.

### EXEMPLES

### EXEMPLE 1 : Procédé de préparation d'un collagène à pouvoir polymérisant (Variante 1 : extrait non pepsiné).

On transvase l'extrait de collagène à 10 %, préparé à partir de tendons d'Achille d'autruche comme ci-dessus, dans le mélangeur à double couteaux transversaux déjà mentionné. On procède alors à une agitation et à un cisaillement à une vitesse de 1500 tours par min et à température ambiante (20 °C) pendant 7 min. On constate une augmentation de la viscosité à 15000 cps. L'extrait prend une coloration blanchâtre et un aspect élastique. On effectue ensuite des dilutions successives de manière à atteindre une concentration de collagène de 6 %. Après chaque dilution, on élève progressivement de 4 °C la température et la vitesse d'agitation de 500 tours par min par intervalle de 3 à 4 min. Lorsqu'on atteint une vitesse d'agitation de 3500 tours par min et 35-36 °C, on maintient ces conditions pendant 3 min environ puis on arrête d'agiter et laisse reposer à la même température de 35-36 °C pendant 30 min. On procède alors à une nouvelle dilution (au 1/5) et on augmente la vitesse pour atteindre 5000 tours par min, et la température pour atteindre 42 °C en 3 à 4 min. Le collagène est alors pâteux et présente une viscosité de 17000-20000 cps (17-20Pa.s). On procède à une augmentation de la température pour atteindre 44 °C. Puis on maintient ces conditions pendant 30 s à 1 min, le collagène pâteux se liquéfie rapidement, prenant un aspect fluide sa viscosité étant réduite à 30-50 cps (30.10⁻³Pa.s à 50.10⁻³Pa.s).

L'extrait liquéfié est transvasé immédiatement dans un dispositif de filtration sous pression préalablement stérilisé à la chaleur et équipé d'une double enveloppe avec circulation d'eau permettant le maintien de la température à 42-45 °C. L'extrait est alors filtré à travers deux membranes stériles de filtration, la première de porosité 0,45 µ et la deuxième de porosité 0,22 µ en profondeur. Le filtrat est recueilli dans un récipient sous pression équipé d'une double enveloppe, la température étant maintenue à 42-45 °C. Ce filtrat est soumis ensuite à une filtration absolue sur membrane de porosité 0,22µ.

Le collagène obtenu est stérile, comme confirmé par les tests bactériologiques selon la Pharmacopée Européenne.

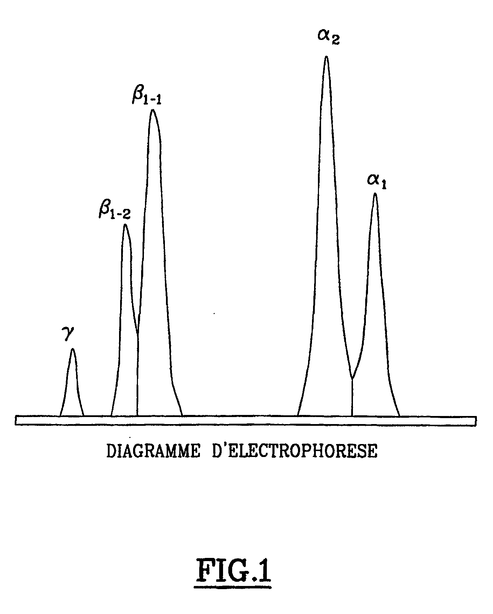

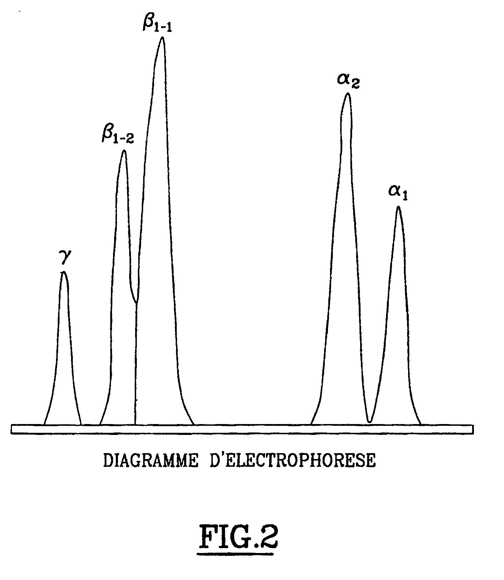

Par ailleurs, les analyses réalisées avant, en cours et après les traitements mécaniques et thermiques du collagène ainsi obtenu montrent que la composition en acides aminés du collagène. demeure inchangée. Par ailleurs on ne détecte pas de polypeptide de poids moléculaire inférieur à 95 000 Da par électrophorèse sur gel de polyacrylamide (Figures 1 et 2 ). On note en outre que le rapport α₂(I)₁/α₁(I)₂ est de 0,49, soit un collagène, à plus de 95 % de type I.

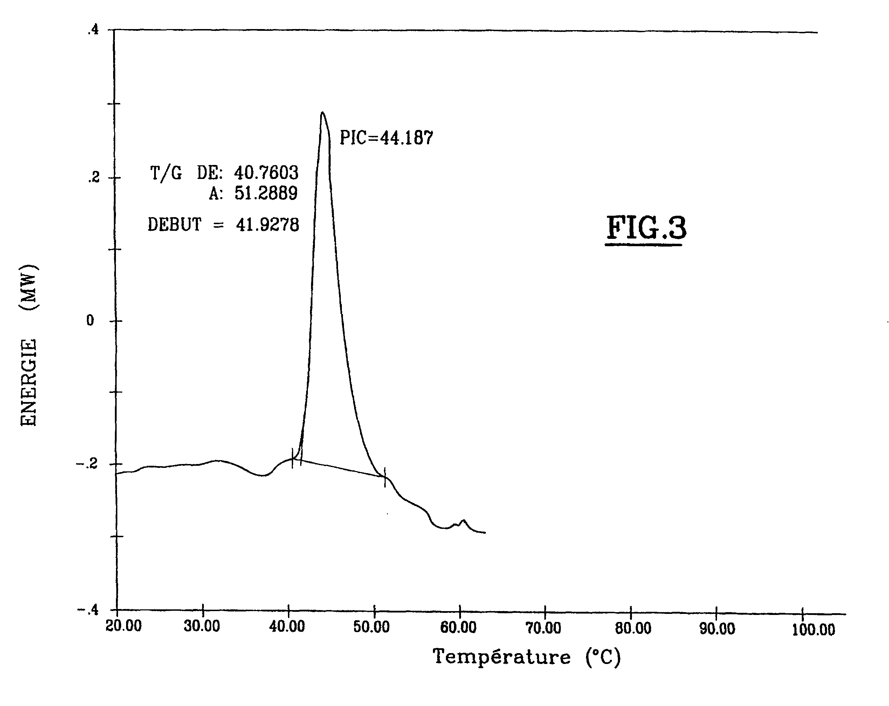

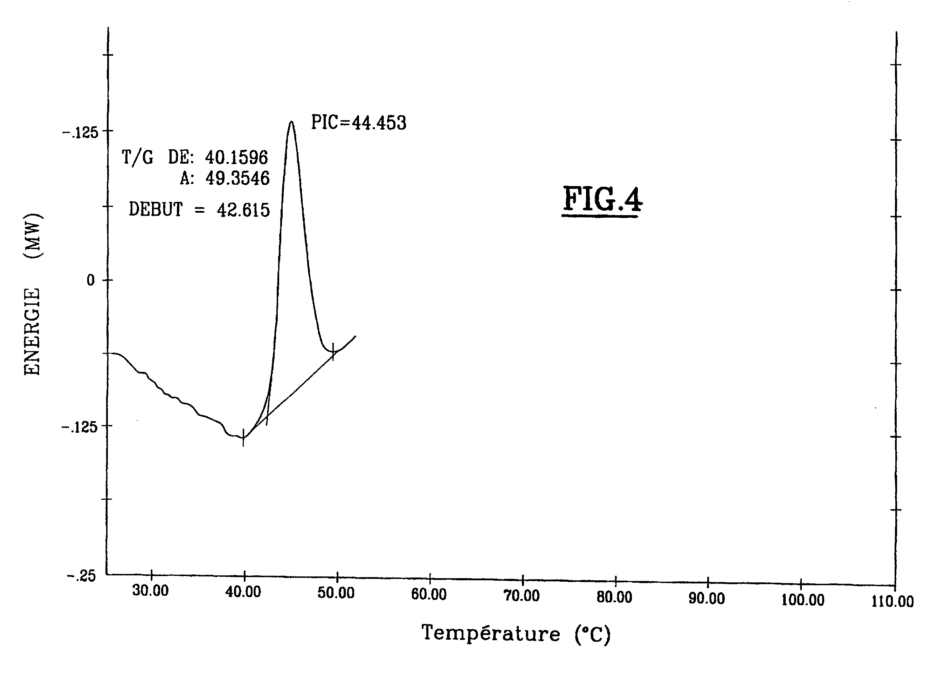

L'analyse thermographique, par thermographie différentielle (DSC) réalisée à l'aide du Modèle DSC 7 Delta-Series de la société Perkin-Elmer, et effectuée au cours du traitement thermique et mécanique à 40-50 °C, confirme la disparition du pic de dénaturation du collagène correspondant à une perte de la structure trihélicoïdale de la molécule. Cependant cette disparition n'est que temporaire puisqu'on constate la réapparition du pic de dénaturation après refroidissement de l'extrait collagénique et sa prise en masse (Figures 3 et 4). C'est la preuve que la perte de la structure trihélicoïdale du collagène au cours du traitement thermique et mécanique conforme à la présente invention est de nature réversible.

L'ensemble de ces résultats confirme à l'évidence le maintien des structures secondaire et tertiaire du collagène ainsi préparé.

Les autres principales caractéristiques et propriétés du collagène ainsi obtenu sont les suivantes :
- taux d'humidité : 95 %
- aspect : liquide visqueux à une température supérieure à 38 °C environ et collant au toucher ; masse gélifiée homogène et compacte pseudo-solide à une température inférieure à 38 °C environ.
- viscosité : 45 cps (45.10⁻³Pa.s) à l'état liquide
- Azote total : 17,9 %
- Hydroxyproline : 12,8 %
- dépourvu de tryptophane, d'aminoglycanes, et de chaînes polypeptidiques < 95000 Dalton.
- pH de 5,5
- lipides < 1 %
- cendres sulfuriques < 2 %

Le collagène stérile préparé, comme il vient d'être décrit, est maintenu à 42-45 °C pendant une période de temps limitée pour être rapidement réparti tel quel dans des récipients ou conditionnements appropriés de type flacons, seringues, blisters etc., ou après mélange à d'autres principes actifs, tels que des facteurs de la coagulation, préalablement stérilisés si besoin.

Utilisé seul, le collagène ainsi produit forme une colle qui présente d'excellentes propriétés hémostatiques pour applications en chirurgie générale, parenchymateuse, cardio-vasculaire, thoracique, orthopédique, ORL, urologique, hépatique, viscérale, gynéco-obstétrique, chirurgie de transplantation et de la moelle osseuse ; en microchirurgie et tout autre domaine médical nécessitant l'action de cette forme médicamenteuse associée ou non à d'autres principes actifs.

Il est conservé à température ambiante où il se prend en masse.

Ceci le distingue nettement des colles dites biologiques actuellement disponibles sur le marché comme Tissucol® de la société Immuno-AG, Autriche, ou Biocolle® des Laboratoires des fractionnements et des biotechnologies, France, dont la conservation pour la forme sèche est obligatoirement à faible température, de l'ordre de +4 °C.

La colle à base de collagène selon la présente invention doit être reconstituée à l'état liquide avant d'être utilisée. Pour ce faire, un simple chauffage de 2 à 3 min suffit, par exemple à l'étuve ou au bain marie à 40-45 °C, suivie d'une faible agitation manuelle.

Ainsi, comparée aux colles connues précitées, la colle à base de collagène selon l'invention présente outre ses caractéristiques avantageuses, un temps de préparation dix fois moins important environ.

Lorsqu'ils sont présents, les facteurs de la coagulation sont préalablement préparés, avant leur ajout au collagène, de la manière suivante.

On reprend une quantité de poudre de thrombine ou de fibrinogène correspondant à 2 UI / mg et 1 UI / mg de collagène respectivement dans une solution de chlorure de calcium 40 mM (soit 1 ml de collagène pour 50 UI de thrombine) et dans de l'eau pour préparation injectable (PPI). On filtre ensuite séparément sur membrane filtrante absolue de porosité 0,22 µ.

On ajoute ensuite la thrombine ou le fibrinogène au collagène stérile préalablement neutralisé (pH 7,0), comme déjà mentionné plus haut. On homogénéise le mélange et on procède à la répartition.

La répartition est effectuée sous pression, par exemple par une pompe doseuse dans des récipients appropriés notamment des seringues, des flacons en verre, par exemple dans le cas de la préparation de colle biologique ; dans des sachets ou plaquettes blisters thermosoudés comme c'est le cas par exemple des «patch», des films, ou des masques hydratants, ou encore dans des plateaux d'un lyophilisateur pour obtenir une poudre ou une éponge, etc., comme décrits précédemment.

### EXEMPLE 2 : Colle biologique liquide stérile de collagène + thrombine

A partir du produit obtenu à l'Exemple 1 sous forme liquide stérile on prépare dans un flacon en verre une composition contenant 2 ml de collagène à 5 % et une quantité correspondante à 200 UI de thrombine.

Lors de son application sur une surface hémorragique, elle se prend en masse et adhère à la surface tissulaire permettant la fermeture de la lésion ou de l'incision et elle agit comme hémostatique de contact conduisant à l'arrêt du saignement.

### EXEMPLE 3 : Poudre de collagène stérile + fibrinogène

A partir du produit obtenu à l'Exemple 1, et après lyophilisation et broyage du collagène associé au fibrinogène, sous forme de poudre stérile on répartit dans des flacons stériles en polyéthylène 360 mg de collagène renfermant 700 UI de fibrinogène par flacon.

Cette poudre appliquée par pulvérisation sur une surface hémorragique, particulièrement en microchirurgie dans des zones d'accès difficile, permet l'arrêt du saignement par son action hémostatique sur les voies intrinsèques et extrinsèques de la coagulation et adhère à la surface hémorragique.

### EXEMPLE 4 : Eponge de collagène stérile + thrombine

A partir de 35 ml de collagène liquide stérile, présentant une concentration de 1 %, tel qu'obtenu à l'Exemple 1 puis neutralisé, on effectue sa répartition dans des barquettes ou plaquettes blisters de 5 x 7 cm. On procède alors à une lyophilisation puis à la fermeture dans des conditions aseptiques des barquettes ou plaquettes blisters qui sont ensuite conditionnées dans des doubles sachets stériles.

L'éponge ainsi produite permet de recouvrir une zone de 35 cm², et correspond à une quantité de 350 mg de collagène incorporant 700 UI de thrombine.

Cette éponge a une action hémostatique rapide associant l'action du collagène à celle de la thrombine sur les mécanismes de la coagulation et adhère à la surface hémorragique permettant l'exercice d'une action mécanique importante. Son aspect filmogène permet de couvrir d'importantes zones hémorragiques.

### EXEMPLE 5 : «patch» de collagène (pseudo-solide) + métronidazole

Au collagène à 2 % obtenu à l'Exemple 1 à l'état liquide à 42-45 °C et neutralisé comme mentionné précédemment, on ajoute l'agent anti-infectieux métronidazole à l'état liquide, préalablement filtré stérilement, à raison de 0,05 mg/mg de collagène sec.

Après refroidissement, le collagène sous forme de gel pseudo-solide homogène et limpide d'une épaisseur de 1 mm est soumis à une stérilisation par irradiation aux rayons beta, à 2,5 Mrad (25.10⁻⁵Gy), Laboratoires Caric Orsay, France.

On constate de manière surprenante que le gel préparé à partir du collagène conforme à la présente invention « ne casse pas », à savoir ne se sépare pas en deux phases, contrairement à un gel classique subissant le même traitement de stérilisation par irradiation.

Ce « patch » appliqué sur une plaie infectée a une action protectrice et topique, agissant en tant que désinfectant grâce à la présence et à la libération de métronidazole.

### EXEMPLE 6 : Collagène atélopeptidique à pouvoir polymérisant (variante 1)

A partir d'un extrait de collagène purifié provenant de tendon d'Achille d'autruche frais à 10 %, on procède comme suit:
- on dilue le collagène par ajout d'une solution d'acide citrique 10⁻² M à pH 2,5 jusqu'à obtention d'une concentration de collagène de 1 %,
- on ajoute la pepsine - 1/10 poids / poids de collagène sec,
- on laisse agir à température ambiante (20 °C) pendant 7 jours,
- le 7 ième jour on neutralise le collagène à l'hydroxyde de sodium (30 %) afin d'obtenir un pH de 7,2. Le collagène précipite, les protéines non collagéniques et les résidus pepsiniques restant en solution étant éliminés par centrifugation avec un dispositif essorent/décanteur comme par exemple celui commercialisé par la société Rousselet sous la référence RC40,
- on procède à des lavages du culot dans l'eau déminéralisée, suivis d'essorage, le poids d'eau correspondant à 10 fois le poids du culot, puis on obtient un culot de collagène sous forme de fibres humides,
- on répète cette opération à trois reprises dans les mêmes conditions, obtenant ainsi des fibres de collagène ayant un taux d'humidité de 70 %,
- on procède à une solubilisation du culot dans l'acide citrique 10⁻² M, à pH 2,5,
- on homogénéise le mélange jusqu'à solubilisation totale et on obtient un extrait de collagène ayant une concentration de 10 %, limpide et présentant une viscosité de 6000 cps (6Pa.s),
- on transvase l'extrait dans le mélangeur à double couteaux transversaux déjà mentionné et on procède selon l'enseignement de l'Exemple 1, jusqu'à obtention d'un collagène à environ 5 %.

Ce collagène atélopeptidique stérile à pouvoir polymérisant est particulièrement avantageux dans les applications ophtalmologiques, et peut se présenter en forme de lentille, de membranes souples ou de gouttes / collyre comme déjà décrit ci-dessus. Par ailleurs, l'absence de télopeptides potentiellement immunogènes le rend spécialement attrayant.

### EXEMPLE 7 : Mousse de collagène (variante 2)

La mousse est préparée à partir d'un culot de fibres humides de collagène, ayant un taux d'humidité de 70 %, traité selon la variante 2 déjà décrite. Ce culot est homogénéisé et est maintenu pendant 48 h sous agitation douce à +4 °C dans de l'eau purifiée. Après centrifugation, le culot est repris dans une solution d'acide citrique 10⁻²M (pH 2,5), la concentration de collagène étant de 2,5 %. Après un repos de 24 h à 4 °C, le culot est transféré dans le mélangeur à doubles couteaux transversaux. La vitesse d'agitation et de cisaillement ainsi que la température sont progressivement augmentées et on atteint 2000 tours par min et une température de 25 °C. L'extrait est alors filtré sur membrane absolue de porosité 0,22 µ à la température opératoire de 25 °C.

Au collagène stérile ainsi obtenu à 2,5 %, on ajoute le chlorure de benzalkonium à une concentration de 1,5 g/l. Le mélange est soumis à une agitation progressive de 500 tours par min à 2500 tours par min à l'ambiante, l'extrait s'épaissit, prend une coloration blanche et on obtient ainsi la mousse conforme à la présente invention.

La mousse est répartie dans des pompes doseuses de 10 ml et plus équipées éventuellement d'un cathéter permettant son administration dans des zones difficiles d'accès comme c'est souvent le cas en microchirurgie.

Ainsi conservée, cette mousse est stable au stockage à température ambiante pendant au moins 24 mois. En cas de présence de principe actif thermosensible, il est préférable de maintenir la température de stockage de la mousse ainsi produite à 4 -10 °C.

Cette mousse est avantageusement appliquée notamment en chirurgie parenchymateuse, et thoracique. Elle est particulièrement utile dans le maintien des espaces inter-tissulaires et parenchymateux et permet d'éviter les adhérences anatomopathologiques entre les tissus endommagés.

### EXEMPLE 8 : Eponges hémostatiques monocouche ou stratifiée (variante 2)

Les éponges sont produites à partir de collagène obtenu selon la variante 2, comme à l'Exemple 7, stérilisé par filtration absolue sur membrane de porosité 0,22 µ.

Pour obtenir une éponge monocouche associant un facteur de la coagulation, par exemple, on procède comme suit :
- 35 ml lde collagène liquide, à la concentration de 1 %, préalablement mélangé à la thrombine (700 UI) ou au fibrinogène (350 UI) stériles, puis neutralisé, sont répartis dans des blisters de 5 x 7 cm.
- on lyophilise de manière classique et séparément chacune des associations précitées de collagène (les températures de congélation et de résorption étant de -25°C et 35 °C, respectivement).

L'éponge de collagène à pH neutre ainsi obtenue a un aspect cotonneux, légèrement fibreux et est de couleur respectivement blanche et crème en présence de fibrinogène et de thrombine.

Cette éponge a une action hémostatique liée à l'action extrinsèque et intrinsèque du collagène sur les mécanismes de la coagulation, qui est de plus renforcée par l'action hémostatique des facteurs de la coagulation que sont la thrombine et le fibrinogène.

Par ailleurs, le collagène à pH neutre tel qu'obtenu selon la variante 2 étant insoluble, exerce une action mécanique sur la surface tissulaire hémorragique, que ne peuvent exercer les facteurs de la coagulation lorsqu'ils sont utilisés seuls, à l'état lyophilisé.

En outre, et comme déjà mentionné précédemment, dans une telle présentation conforme à la présente invention l'activité des facteurs de la coagulation est entièrement préservée, contrairement aux préparations classiques - tels que, par exemple le Tachocomb® Hafslund Nycomed, décrits dans le document EP-A-59 265 - où ces derniers sont stérilisés aux rayonnement ionisants et donc partiellement dénaturés.

Pour obtenir une éponge stratifiée, on procède comme suit :
- on répartit 18 ml de collagène liquide à pH 5, à la concentration de 0,5 %, et préalablement mélangé à la thrombine (700 UI) et à 1 ml de chlorure de calcium 40 mM, par blister de 5 x 7 cm, puis
- on effectue une congélation douce à -10 °C puis on ajoute à cette première couche 18 ml de collagène liquide à pH 5 à 0,5 % préalablement mélangé au fibrinogène (350 UI), puis
- on procède à une nouvelle congélation douce dans les mêmes conditions et on ajoute à la deuxième couche ainsi obtenue une dernière couche de collagène liquide à 1 % cette fois à pH neutre, et enfin
- on effectue une lyophilisation comme décrit précédemment dans le cas de l'éponge monocouche.

Ainsi on obtient une éponge stratifiée à trois couches aux propriétés remarquables suivantes :
* une fois appliquée, le collagène à pH acide est immédiatement solubilisé libérant ainsi les facteurs de la coagulation qui forment une colle hémostatique sur la surface hémorragique,
* la couche externe à pH neutre exerce en outre une action mécanique renforçant ainsi l'action de la colle hémostatique, d'autant plus que la zone hémorragique est importante.

Par ailleurs, cette éponge conforme à la invention présente l'avantage de ne nécessiter aucune préparation préalable à son emploi.

Ce type de présentation hémostatique est particulièrement indiqué en chirurgie générale, parenchymateuse, cardio-vasculaire, thoracique, plastique, orthopédique, ORL, urologique, hépatique, viscérale, gynéco-obstétrique, chirurgie de la transplantation et de la moelle osseuse, microchirurgie et généralement dans tout domaine nécessitant une telle action hémostatique.

### EXEMPLE 9 : Eponge + vancomycine (variante 2)

On procède de la manière suivante :
- dans un récipient stérile on introduit 6 l d'une solution de collagène à 1,4 %, telle qu'obtenue selon la variante 2 du procédé conforme à la présente invention, et neutralisée par addition d'hydroxyde de sodium, puis
- on ajoute 1 l d'une solution de chlorhydrate de vancomycine à 42 g/l à la solution de collagène maintenue à température ambiante dans l'heure suivant sa neutralisation, puis
- on homogénéise la solution, puis à l'aide d'une pompe doseuse on transvase à travers une membrane filtrante de porosité 0,22 µ le mélange résultant dans des récipients de lyophilisation à raison de 70 ml par récipient de 10 cm de côté, et ensuite
- on débute le cycle de congélation jusqu'à -25 °C suivi du cycle de sublimation et de désorption jusqu'à +35 °C,
- on obtient enfin des éponges stériles d'une épaisseur de 7 mm, contenant du collagène à raison de 8,4 mg de collagène / cm² et 4,2 mg de Chlorhydrate de vancomycine / cm² ou des éponges d'1 à 2 mm d'épaisseur en exerçant une pression automatique sur la surface des couches à l'intérieur du lyophilisateur pour obtenir une éponge laminée, ensuite
- on procède à la thermosoudure des blisters et on conditionne en double sachets de polyéthylène stériles.

Des éponges identiques conformes à l'invention sont obtenues incorporant à la place de la vancomycine, la gentamicine, la péfloxacine ou d'autres antibiotiques ou agents anti-infectieux.

Ces éponges, libérant localement et de manière progressive leur(s) principe(s) actif(s), sont particulièrement utiles dans le traitement de nombreuses pathologies, notamment osseuse, viscérale, traumatologique et chirurgicale en particulier dans la prévention ou le traitement de contaminations microbiennes éventuelles.

Ces éponges de collagène conformes à la présente invention, outre leur excellente tolérance et biodégradabilité, ont le sérieux avantage d'incorporer la vancomycine stérilisée par filtration, comme décrit ci-avant, et ayant donc conservé toute son activité contrairement aux préparations classiques où cet antibiotique est partiellement dénaturé par stérilisation aux rayonnements ionisants.

## Revendications

1. Procédé de préparation d'un collagène à partir d'un extrait solubilisé et purifié, éventuellement pepsiné, de collagène natif non stérile, **caractérisé en ce qu'**il comprend les étapes consistant :
- (i) à agiter et cisailler ledit extrait dans un mélangeur à double couteaux transversaux en augmentant par palier la vitesse d'agitation, sans dépasser 10000 tours par min, et la température de 2°C à 10°C, de préférence 3°C à 5°C, de manière à accroître la température ambiante initiale jusqu'à une température maximale contrôlée, de préférence inférieure à 50 °C, puis
- (ii) à stériliser en milieu liquide ledit extrait, ce par quoi on obtient un collagène stérile à l'état natif ou atélopeptidique.

2. Procédé selon la revendication 1, l'extrait solubilisé et purifié n'étant pas pepsiné, **caractérisé en ce qu'**à l'étape (i) on effectue une dilution de l'extrait à chacun desdits paliers où la viscosité dudit extrait atteint une valeur élevée choisie de 15-20Pa.s et, lorsque ladite température maximale est de 42-44 °C, on laisse reposer ledit extrait juste avant l'étape (ii).

3. Procédé selon la revendication 1 ou 2, **caractérisé en ce qu'**avant l'étape (ii) ledit extrait est filtré en profondeur à ladite température maximale contrôlée sur membranes de porosité 0,45 µ-0,22 µ.

4. Procédé selon la revendication 1, ledit extrait solubilisé et purifié étant pepsiné, **caractérisé en ce qu'**on traite ledit extrait comme suit :
- on réalise des filtrations clarifiantes « en cascade » sur membranes de différentes porosité choisies, jusqu'à 0,45 µ, puis
- on effectue une précipitation différentielle au chlorure de sodium du filtrat,
et **en ce qu'** à l'étape (i) la température maximale atteinte est de 35°C, de préférence 25 °C, et la vitesse d'agitation est de 1000-5000 tours par min, de préférence 2500 tours par min.

5. Procédé selon la revendication 3 ou 4, **caractérisé en ce qu'**à l'étape (ii) on effectue une filtration absolue sur membrane de porosité 0,22 µ ou on stérilise l'extrait par ajout d'acide peracétique.

6. Procédé selon l'une quelconque des revendications précédentes **caractérisé en ce qu'**il comprend une étape de neutralisation dudit extrait avant ou après l'étape (ii).

7. Procédé selon l'une quelconque des revendications précédentes **caractérisé en ce que** le collagène est de type fibrillaire, de préférence un collagène majoritairement de type I.

8. Procédé selon l'une quelconque des revendications précédentes **caractérisé en ce que** l'extrait de collagène est obtenu à partir de dermes, de tissus conjonctifs ou de tendons animaux, notamment dermes de lapins et tendons d'Achille d'autruche.

9. Collagène natif ou atélopeptidique de type I, susceptible d'être obtenu par un procédé selon l'une quelconque des revendication 5 à 8, **caractérisé en ce qu'**il présente les caractéristiques ou propriétés suivantes :
- rapport α₂(I)₁/α₁(I)₂ de 0,48 à 0,52
- stérile selon la norme de la pharmacopée Européenne
- Azote total : 17,0 % à 18,7 %
- Hydroxyproline : de 12 % à 13,9 %
- dépourvu de tryptophane, d'aminoglycanes et de polypeptides de poids moléculaire < 95000 Dalton
- lipides < 1 %
- cendres sulfuriques < 2 %

10. Composition pharmaceutique et/ou para-pharmaceutique et/ou médico-chirurgicale et/ou ophtalmologique et/ou cosmétique comprenant un collagène selon la revendication 9 seul ou associé à au moins un autre principe actif.

11. Composition selon la revendication précédente **caractérisé en ce que** ledit autre principe actif est choisi dans le groupe comprenant les facteurs de la coagulation, par exemple la thrombine, les agents anti-infectieux, notamment le métronidazole, les antibiotiques notamment les macrolides tels que la gentamicine, les glycopeptides tels que la vancomycine, les fluoroquinolones tels que la péfloxacine, les anti-inflammatoires stéroïdiens et non stéroïdiens acylcarboxyliques ou dérivés oxicam, les facteurs de croissance, notamment osseux, tels que la somatotropine, et épidermiques, tels que EGF (facteur de croissance épidermique) les tensioactifs, les agents hydratant, les agents stabilisant, et les vitamines.

12. Présentation galénique d'une composition selon la revendication 10 ou 11, **caractérisée en ce qu'**elle est choisie dans le groupe comprenant les gels, notamment les gels injectables et les gels pseudo-solides, les mousses, les collyres, les éponges monocouche ou stratifiées, les poudres, les plaques ou bandeaux, les masques, les spray, les films, les membranes, les feuilles, et les fils, en particulier, pour sutures.

13. Utilisation d'un collagène selon la revendication 9 dans la fabrication. d'une composition destinée à un traitement pharmaceutique et/ou para-pharmaceutique et/ou médico-chirurgical et/ou ophtalmologique d'un corps humain ou animal.

## Claims

1. A method for preparing a collagen from a solubilized and purified, optionally pepsinized, extract of nonsterile native collagen, **characterized in that** it comprises the steps consisting:
- (i) in stirring and shearing said extract in a mixer with double transverse cutters while increasing in stages the stirring speed, without exceeding 10,000 rpm, and the temperature from 2°C to 10°C, preferably 3°C to 5°C, so as to increase the initial ambient temperature up to a. controlled maximum temperature, preferably lower than 50°C, and then
- (ii) in sterilizing in liquid medium said extract, as a result of which a sterile collagen in native or atelopeptide form is obtained.

2. The method as claimed in claim 1, the solubilized and purified extract not being pepsinized, **characterized in that**, in step (i), a dilution of the extract is carried out at each of said stages at which the viscosity of said extract reaches a chosen high value of 15 to 20 Pa.s, and, when said maximum temperature is 42 to 44°C, said extract is left to stand just before step (ii).

3. The method as claimed in either of claims 1 and 2, **characterized in that**, before step (ii), said extract is thoroughly filtered at said controled maximum temperature through membranes with a porosity of 0.45 µ to 0.22 µ.

4. The method as claimed in claim 1, said solubilized and purified extract being pepsinized, **characterized in that** said extract is treated as follows:
- clarifying filtrations are carried out in cascade through membranes of various chosen porosities, up to 0.45 µ, and then
- differential precipitation of the filtrate is carried out with sodium chloride,
and **in that**, in step (i), the maximum temperature reached is 35°C, preferably 25°C, and the stirring speed is 1000 to 5000 rpm, preferably 2500 rpm.

5. The method as claimed in either of claims 3 and 4, **characterized in that**, in step (ii), an absolute filtration is carried out through a membrane with a porosity of 0.22 µ, or the extract is sterilized by adding peracetic acid.

6. The method as claimed in any of one of the preceding claims, **characterized in that** it comprises a step of neutralization of said extract before or after step (ii).

7. The method as claimed in any one of the preceding claims, **characterized in that** the collagen is of fibrillar type, preferably a collagen mostly of type I.

8. The method as claimed in any one of the preceding claims, **characterized in that** the extract of collagen is obtained from dermides, from connective tissues or from animal tendons, in particular rabbit dermides and ostrich Achilles tendons.

9. Native or atelopeptide type I collagen, which can be obtained by a method as claimed in any one of claims 5 to 8, **characterized in that** it has the following characteristics or properties:
- α₂(I)₁/α₁(I)₂ ratio of 0.48 to 0.52
- sterile according to the standard of the European Pharmacopeia
- total nitrogen: 17.0% to 18.7%
- hydroxyproline: from 12% to 13.9%
- free of tryptophan, aminoglycans and polypeptides of molecular weight <95,000 daltons
- lipids < 1%
- sulfuric ash < 2%

10. A pharmaceutical and/or parapharmaceutical and/or medico-surgical and/or ophthalmological and/or cosmetic composition comprising a collagen as claimed in claim 9 on its own or combined with at least one other active principle.

11. The composition as claimed in the preceding claim, **characterized in that** said other active principle is chosen from the group comprising clotting factors, for example thrombin, anti-infectious agents, in particular metronidazole, antibiotics, in particular macrolides such as gentamicin, glycopeptides such as vancomycin, and fluoroquinolones such as pefloxacin, acylcarboxylic or oxicam derivative steroidal and nonsteroidal anti-inflammatory agents, growth factors, in particular bone growth factors such as somatotropin, and epidermal growth factors such as EGF (epidermal growth factor), surfactants, moisturizers, stabilizers and vitamins.

12. A pharmaceutical presentation of a composition as claimed in either of claims 10 and 11, **characterized in that** it is chosen from the group comprising gels, in particular injectable gels and pseudosolid gels, foams, eyewashes, monolayer or stratified sponges, powders, plates or bands, masks, sprays, films, membranes, sheets and threads, in particular for sutures.

13. The use of a collagen as claimed in claim 9 in the manufacture of a composition intended for pharmaceutical and/or parapharmaceutical and/or medico-surgical and/or ophthalmological treatment of a human or animal body.

## Patentansprüche

1. Vorbereitungsverfahren eines Kollagen aus einem verlösten, gereinigten Extrakt, eventuell pepsiniert, aus einem reinen , keimfreien Kollagen , durch nachstehende Etappen **gekennzeichnet** :
- (i) Diesen Extrakt in einem Mischer mit Doppel-Querschneider schütteln und ziselieren, wobei die Schüttelgeschwindigkeit stufenweise erhöht wird, ohne 10000 Drehungen/Min. und die Temperatur von 2°C bis 10°C zu überschreiten , vorzugsweise 3°C bis 5°C, sodass die ursprüngliche Raumtemperatur bis zu einer kontrollierten maximalen Temperatur erhöht wird, am besten unter 50°C, dann
- (ii) Den Extrakt in einer flüssigen Umwelt zu sterilisieren, wodurch man einen sterilen keimfreien oder atelopeptidischen Kollagen gewinnt.

2. Verfahren nach der Forderung 1. Der verlöste und gereinigte Extrakt aber nicht pepsinierte, **dadurch gekennzeichnet, dass** man in der Etappe (i) eine Verdünnung des Extrakts in jeder genannten Stufen durchführt, wobei die Viskosität dieses Extraktes einen gewählten hohen Wert von 15-20Pa.s erreicht und wenn die höchste Temperatur 42-44 °C erreicht, lässt man den Extrakt ruhen knapp vor der Etappe (ii).

3. Verfahren nach der Forderung 1 und 2, **dadurch gekennzeichnet, dass** vor der Etappe (ii) dieser Extrakt in der Tiefe bei der besagten kontrollierten maximalen Temperatur auf Porositätsmembranen 0,45 µ-0,22 µ gefiltert wird.

4. Verfahren nach der Forderung 1, Der verlöste und gereinigte und pepsinierte Extrakt, **dadurch gekennzeichnet, dass** man den Extrakt wie nachstehend behandelt :
- Man führt "serienweise" klärenden Filtrationen auf Membranen mit ausgesuchten unterschiedlichen Porositäten bis 0,45 µ durch, dann:
- Man führt eine Differentialpräzipitation mit dem Natriumchlorid des Filtrats.
Bei der Etappe (i) ist die erreichte Temperatur 35°C, am besten 25°C, die Geschwindigkeit des Schüttelns beträgt 1000-5000 Drehungen in der Minute, am besten 2500 Drehungen in der Minute.

5. Verfahren nach der Forderung 3 oder 4, **dadurch gekennzeichnet, dass** man bei der Etappe (ii) eine absolute Filtration auf Porositätsmembranen 0,22 µ. durchführt oder den Extrakt durch Zusatz von Essigsäure sterilisiert.

6. Verfahren aufgrund einer der vorhergehenden Forderungen 3 oder 4, **dadurch gekennzeichnet , dass** es eine Neutralisationsetappe des besagten Extrakts vor oder nach der Etappe (ii) enthält.

7. Verfahren aufgrund einer der vorhergehenden Forderungen, **dadurch gekennzeichnet , dass** das Kollagen vom Typ faserig ist, vorzugsweise ein Kollagen mehrheitlich vom Typ I ist.

8. Verfahren aufgrund einer der vorhergehenden Forderungen, **dadurch gekennzeichnet , dass** der Extrakt des Kollagen aus den Lederhäuten, Bindegeweben, oder aus den Tiersehnen, vor allem aus den Lederhäuten der Kaninchen und den Achillessehnen der Sträuße gewonnen wird.

9. Keimfreies oder atelopeptidisches Kollagen des Typ I, könnte durch ein Verfahren nach einer der Forderungen 5 bis 8 gewonnen werden, **dadurch gekennzeichnet, dass** es folgende Eigenschaften und Funktionen darstellt:
- Bericht a2 (I) √a1 (I)2 von 0,48 bis 0,52
- Steril, gemäss der europäischen Pharmakopie
- Gesamter Stickstoff: 17,0 % bis 18,7 %
- Hydroxyproline : von 12 % bis 13,9 %
- Frei von Triptophahn, Aminoglykan und von Polypeptiden eines molekularen < 95000 Dalton.
- Fettstoffe <1 %
- Schwefelasche

10. Pharmazeutische und/oder para-pharmazeutische und/oder chirurgie-medizinische und/oder kosmetische Zusammenstellung enthalten ein alleiniges Kollagen oder mindestens in Vereinigung mit einem anderen Wirkstoff, gemäss Forderung 9.

11. Zusammenstellung nach der vorherigen Forderung, **dadurch gekennzeichnet, dass** der andere Wirkstoff unter der Gruppe der Gerinnungsfaktoren gewählt wird, wie zum Beispiel die Thrombin, Antiinfektionsstoffe, vor allem die Makroliden, nämlich die Gentamizin, Glykolpeptiden, wie die Vankomyzin, die Fluoroquinolonen wie die Pefloxanin, die steroiden und nicht steroiden Anti-Entzünder, Azylcarboxylen oder Oxicam Derivate, Entwicklungsfaktoren, vor allem knochenartige, wie die Somatotropin, und epidermale, wie EGF (Epidermaler Entwicklungsfaktor), die Tensio-wirkstoffe, Feuchtigkeitswirkstoffe, die stabilisierenden Wirkstoffe und Vitaminen.

12. Galenische Darstellung einer Zusammenstellung gemäss Forderung 10 oder 11, gewählt aus der Gruppe der Gel, vor allem die einspritzenden Gels und die pseudo-harten Gels, die Schäume, die Kollyren, die einschichtigen oder mehrschichtigen Schwämme, Puder, Belagen und Binden, Masken, Spray, Filme, Membranen, Blätter, Fäden, und vor allem für Nähte

13. Verwendung eines Kollagen gemäss Forderung 9 in der Herstellung einer Zusammenstellung, die für die Pharmazeutische und/oder para-pharmazeutische und/oder chirurgiemedizinische und/oder ophtalmologische Behandlung eines Menschen oder Tierkörpers bestimmt ist.
